# EUROPEAN PATENT APPLICATION

(11) **EP 4 098 740 A1**
(43) Date of publication of application: **07.12.2022**
(21) Application number: 21754401.4
(22) Date of filing: 15.02.2021
(51) Int. Cl.: C12N 5/077, C12N 5/071, C12M 3/00

(54) **DECELLULARIZED HEART EXTRACELLULAR MATRIX-DERIVED SCAFFOLD FOR CULTURING AND TRANSPLANTING HEART ORGANOID, AND PREPARATION METHOD THEREFOR**

(30) Priority: 14.02.2020 KR 20200018365
(71) Applicant: Cellartgen Inc., Seoul 03722 (KR); Republic of Korea (Ministy of Food and Drug Safety), Chungcheongbuk-do 28159 (KR)
(72) Inventor: CHO, Seung Woo, Seoul 03722 (KR); MIN, Sung Jin, Seoul 03722 (KR); CHOI, Yi Sun, Seoul 03722 (KR); KIM, Su Ran, Seoul 03722 (KR)
(74) Representative: Manitz Finsterwald Patent- und Rechtsanwaltspartnerschaft mbB
(86) International application number: PCT/KR2021/001924
(87) International publication number: WO 2021/162530

(57) **Abstract**

The present disclosure relates to a scaffold for culturing and transplanting a cardiac organoid by using a heart extracellular matrix (HEM).

## Description

### TECHNICAL FIELD

The present disclosure relates to a heart extracellular matrix-derived scaffold for culturing and transplanting a cardiac organoid and a method of preparing the same.

### BACKGROUND

An organoid is a three-dimensional structure composed of various cells constituting the tissue and thus can mimic the environment of living tissue. Therefore, the organoid has recently attracted a great deal of interest. For this reason, it is widely used in various application fields, such as new drug development, disease modeling and regenerative therapy, as well as basic biology field.

Currently, most of disease models for new drug development are animal models. Since animals are different from humans in terms of genetics and physiology, there are limits to mimicking human diseases in animals. Also, ethical issues with animal testing constantly emerge. When an organoid model made from patient-derived cells is used, it is possible to research the mechanism of disease as well as to provide customized diagnosis and treatment to patients. Therefore, the organoid model has recently attracted a great deal of interest.

Currently, various types of organoid models derived from stem cells of various organs in vivo have been constructed. When culturing these various types of organoids, most of researchers around the world use Matrigel as a culture scaffold. However, Matrigel is a component derived from a mouse sarcoma line, and concerns are raised about attempts to transplant organoids cultured in Matrigel into humans due to the risk of infection and immune rejection. Also, Matrigel is not an extracellular matrix component included in actual tissues, and, thus, it cannot implement a tissue-specific extracellular matrix microenvironment essential for cell growth and differentiation. Therefore, there is an urgent need for development of a biocompatible and tissue-specific organoid culture scaffold that can solve the problems of Matrigel.

The present disclosure proposes a novel culture platform that produces a matrix containing only an extracellular matrix component from the cardiac tissue through decellularization and uses the matrix for cardiac organoid culture. The decellularized heart extracellular matrix-derived scaffold contains various extracellular matrix components and growth factors present in the cardiac tissue and thus provides a cardiac tissue-specific microenvironment. Thus, it is expected to promote formation, growth and differentiation of cardiac organoids. Mature cardiac organoids prepared in this way are expected to be used as a platform for cardiac disease modeling and drug toxicity evaluation as well as to be applied to the field of regenerative medicine.

### DISCLOSURE OF THE INVENTION

### PROBLEMS TO BE SOLVED BY THE INVENTION

The present disclosure is conceived to prepare a large amount of heart extracellular matrix-derived scaffolds through a series of chemical treatments on a porcine cardiac tissue and to apply them to culturing of a cardiac organoid.

However, the problems to be solved by the present disclosure are not limited to the above-described problems. Although not described herein, other problems to be solved by the present disclosure can be clearly understood by a person with ordinary skill in the art from the following descriptions.

### MEANS FOR SOLVING THE PROBLEMS

Hereafter, the present disclosure will be described in detail with reference to the accompanying drawings. However, it is to be noted that the present disclosure is not limited to examples described herein but can be embodied in various other ways. It is to be understood that the term "comprises or includes" and/or "comprising or including" used in the document means that one or more other components, steps, operation and/or existence or addition of elements are not excluded in addition to the described components, steps, operation and/or elements unless context dictates otherwise.

Unless otherwise indicated, the practice of the disclosure involves conventional techniques commonly used in molecular biology, microbiology, protein purification, protein engineering, protein and DNA sequencing, and recombinant DNA fields, which are within the skill of the art. Such techniques are known to a person with ordinary skill in the art and are described in numerous standard texts and reference works.

Unless defined otherwise herein, all technical and scientific terms used herein have the same meaning as commonly understood by a person with ordinary skill in the art to which this disclosure belongs.

Various scientific dictionaries that include the terms included herein are well known and available to those in the art. Although any methods and materials similar or equivalent to those described herein find use in the practice or testing of the disclosure, some preferred methods and materials are described. It is to be understood that this disclosure is not limited to the particular methodology, protocols, and reagents described, as these may vary, depending upon the context in which they are used by a person with ordinary skill in the art. Hereinafter, the present disclosure will be described in more detail.

One aspect of the present disclosure provides a scaffold composition containing a heart extracellular matrix (HEM).

The term "extracellular matrix" refers to a natural scaffold for cell growth that is prepared through decellularization of tissue found in mammals and multicellular organisms. The extracellular matrix can be further processed through dialysis or crosslinking.

The extracellular matrix may be a mixture of structural or non-structural biomolecules including, but not limited to, collagens, elastins, laminins, glycosaminoglycans, proteoglycans, antimicrobials, chemoattractants, cytokines and growth factors.

In mammals, the extracellular matrix may contain about 90% collagen in various forms. The extracellular matrices derived from various living tissues may differ in their overall structure and composition due to the unique role needed for each tissue.

The term "derive" or "derived" refers to a component obtained from any stated source by any useful method.

Further, in an embodiment of the present disclosure, the heart extracellular matrix may be contained in a concentration of from 0.01 mg/mL to 10 mg/mL, specifically from 0.5 mg/mL to 9 mg/mL, more specifically, from 1 mg/mL to 8 mg/mL, and most specifically, 2 mg/mL, 4 mg/mL or 6 mg/mL. In an optimized embodiment, the heart extracellular matrix may be contained in a concentration of 2 mg/mL. The heart extracellular matrix contained in a concentration out of the above-described range may make it impossible to achieve the intended effect of the present disclosure, or may be unsuitable for preparation or application.

In an embodiment of the present disclosure, the composition may have an elastic modulus of from 1 Pa to 150 Pa at 0.1 Hz to 10 Hz. When the composition has an elastic modulus in the above-described range, a stable polymer network can be formed.

The scaffold composition includes a three-dimensional culture hydrogel prepared based on a cardiac tissue matrix composition obtained by decellularization, and can be effectively used for culturing a cardiac organoid or cardiac single cells.

The decellularized cardiac tissue contains actual tissue-specific extracellular matrix components and thus can provide the physical, mechanical and biochemical environment of the tissue, and is highly efficient in enhancing differentiation into cardiac tissue cells and tissue-specific functionality.

The term "organoid" refers to an ultraminiature body organ prepared in the form of an artificial organ by culturing cells derived from tissues or pluripotent stem cells in a 3D form.

The organoid is a three-dimensional tissue analog that contains organ-specific cells which originate from stem cells and self-organize (or self-pattern) in a similar manner to the in vivo condition. The organoid can be developed into a specific tissue by patterning a restricted element (for example, a growth factor).

The organoid can have the original physiological characteristics of the cells and can have an anatomical structure that mimics the original state of a cell mixture (including all remaining stem cells and the neighboring physiological niche as well as limited cell types). A three-dimensional culture method allows the organoid to be better arranged for improved cellular function, and to have an organ-like form with functionality and a tissue-specific function.

The composition may be formed for encapsulating a cardiac organoid or single cardiomyocytes. Specifically, single cardiomyocytes may be encapsulated and cultured to produce a cardiac organoid, or a cultured cardiac organoid may be encapsulated. When the composition encapsulates single cardiomyocytes, it may further include other cells to reflect the in vivo microenvironment. Specifically, the composition may further include vascular cells and cardiac fibroblasts. The composition may encapsulate a cardiac organoid or single cardiomyocytes to enhance myocardial differentiation.

Another aspect of the present disclosure provides a method of preparing a scaffold composition, including: a process (a) of decellularizing an isolated cardiac tissue to prepare a decellularized cardiac tissue; and a process (b) of drying the decellularized cardiac tissue to prepare a heart extracellular matrix (HEM).

The process (a) is a process of preparing a decellularized cardiac tissue by decellularizing an isolated cardiac tissue.

In an embodiment of the present disclosure, in the process (a), the isolated cardiac tissue is subjected to treatment with 0.1% to 2% sodium dodecyl sulfate (SDS) at 1°C to 10°C for 12 hours to 36 hours, specifically with 0.5% to 1.5% sodium dodecyl sulfate (SDS) at 2°C to 9°C for 15 hours to 30 hours, more specifically with 1% sodium dodecyl sulfate (SDS) at 4°C for 24 hours and decellularized with stirring in a decellularization solution. The overall decellularization efficiency can be increased by adding this treatment prior to stirring in the decellularization solution.

The decellularization solution may include various components for removing cells from the cardiac tissue, and may include, for example, hypertonic saline, peracetic acid, Triton X-100, SDS or other detergent components. In an embodiment of the present disclosure, the decellularization solution may include 0.1% to 5% Triton X-100 and 0.01% to 0.5% ammonium hydroxide and more specifically 1% Triton X-100 and 0.1% ammonium hydroxide. By using the decellularization solution as described above, it is possible to effectively remove DNA in the scaffold prepared by decellularization under more mild conditions than those in the conventional process and also possible to preserve more various proteins in the cardiac tissue.

The stirring may be performed for 3 hours to 24 hours, more specifically 4 hours to 12 hours and most specifically 5 hours to 8 hours. The stirring may be performed for, for example, 6 hours. The stirring may be performed at 1°C to 10°C, specifically 2°C to 9°C and most specifically 4°C. Through this stirring process (decellularization), 95% to 99.9%, more specifically 96% to 98%, of cardiac tissue cells may be removed. When decellularization is performed at a time outside the above-described range or at a level of removal of cardiac tissue cells, the quality of the prepared scaffold composition may be degraded or the economic efficiency of the process may be decreased.

The process (b) is a process of preparing a heart extracellular matrix (HEM) by drying the decellularized cardiac tissue.

The decellularized cardiac tissue may be dried by a known method. The decellularized cardiac tissue may be naturally dried or lyophilized. After drying, the decellularized cardiac tissue may be exposed to ethylene oxide gas or supercritical carbon dioxide by electron beam or gamma radiation for sterilization.

In an embodiment of the present disclosure, after the process (b), the heart extracellular matrix may be contained in a concentration of from 0.01 mg/mLto 10 mg/mL, specifically from 0.5 mg/mL to 9 mg/mL, more specifically, from 1 mg/mL to 8 mg/mL, and most specifically, 2 mg/mL, 4 mg/mL or 6 mg/mL. In an optimized embodiment, the heart extracellular matrix may be contained in a concentration of 2 mg/mL. The heart extracellular matrix contained in a concentration out of the above-described range may make it impossible to achieve the intended effect of the present disclosure, or may be unsuitable for preparation or application.

The dried extracellular matrix may be comminuted by methods including tearing, milling, cutting, grinding and shearing. The comminuted extracellular matrix may be processed into a powder form by a method such as grinding or milling in a frozen or lyophilized state.

The method of preparing a scaffold composition may further include a process (c) of gelating the dried heart extracellular matrix.

The process (c) is a process of gelating the dried heart extracellular matrix.

Through the gelation, the heart extracellular matrix may be crosslinked to prepare a three-dimensional hydrogel-type scaffold, and the gelated scaffold can be used in various fields related to tests, screening and organoid culture.

The term "hydrogel" is a material in which a liquid that contains water as a dispersion medium is hardened through a sol-gel phase transition to lose fluidity and to form a porous structure. The hydrogel can be formed by causing a hydrophilic polymer that has a three-dimensional network structure and a microcrystalline structure to contain water and to be expanded.

The gelation may be performed by dissolving the heart extracellular matrix in an acidic solution with a protease such as pepsin or trypsin at a neutral pH in an electrolyte state of 1X PBS buffer by using 10X PBS and 1 M NaOH at a temperature of 37°C for 30 minutes.

A cardiac organoid or single cardiomyocytes may be encapsulated through the gelation. Specifically, single cardiomyocytes may be encapsulated through the gelation and cultured to produce a cardiac organoid, or a cultured cardiac organoid may be encapsulated. When the composition encapsulates single cardiomyocytes, other cells may be further included to reflect the in vivo microenvironment. Specifically, vascular cells and cardiac fibroblasts may be further included. The composition may encapsulate a cardiac organoid or single cardiomyocytes to enhance myocardial differentiation.

Yet another aspect of the present disclosure provides a method of culturing the scaffold composition or a cardiac organoid prepared in the scaffold composition prepared by the preparation method.

The existing Matrigel-based culture system is an extract derived from animal cancer tissue, has a large difference between the batches, does not simulate the actual cardiac environment, and exhibits insufficient efficiency in differentiation or development into a cardiac organoid. The scaffold composition can create a cardiac tissue-like environment and thus is suitable for cardiac organoid culture.

Specifically, the scaffold composition or a scaffold composition prepared by the above-described preparation method may be used to culture a cardiac organoid by encapsulating a cardiac organoid or single cardiomyocytes. More specifically, single cardiomyocytes may be encapsulated and cultured to culture a cardiac organoid, or a cultured cardiac organoid may be encapsulated and cultured. When the scaffold composition encapsulates single cardiomyocytes, it may further include other cells to reflect the in vivo microenvironment. Specifically, the scaffold composition may further include vascular cells and cardiac fibroblasts. The composition may encapsulate a cardiac organoid or single cardiomyocytes to enhance myocardial differentiation.

The culture refers to a process of maintaining and growing cells under suitable conditions, and the suitable conditions may refer to, for example, the temperature, nutrient availability, atmospheric CO₂ level and cell density at which the cells are maintained.

Appropriate culture conditions for maintaining, proliferating, expanding and differentiating different types of cells are shown in the art and are documented. Suitable conditions for formation of the organoid may facilitate or allow cell differentiation and formation of a multicellular structure.

### EFFECTS OF THE INVENTION

The heart extracellular matrix-derived scaffold prepared in the present disclosure makes it possible to implement a cardiac microenvironment by the cardiac tissue-specific extracellular matrix component abundantly present in the scaffold. Thus, it is possible to prepare a more advanced organoid that mimics the actual cardiac tissue. This can replace Matrigel, which is a representative commercially available organoid culture scaffold, and can create a high value-added industry.

Also, a human cardiac organoid advanced in structure and function has high potential for use in next-generation new drug development and drug toxicity evaluation. Accurate prediction of the cardiac toxicity of drugs during the new drug development process is one of the important success factors for new drug development. With existing cell lines or animal models, the cardiac toxicity of drugs cannot be accurately predicted in many cases. Thus, a lot of money and time are wasted in new drug development. The human cardiac organoid provides a higher accuracy in determining the efficacy and safety of a drug than existing models and is economical in terms of time and cost. Therefore, the human cardiac organoid is expected to make a great contribution to the pharmaceutical industry for new drug development.

Since the human cardiac organoid constructed in the present disclosure can structurally and functionally mimic the cardiac tissue with high precision, it can be used for production of various cardiac disease models that are currently difficult to implement. Therefore, a cardiac disease modeling platform adopting the heart extracellular matrix-derived scaffold-based organoid culture technology can be applied to research the mechanisms of intractable cardiac diseases and thus can contribute to the development of the medical industry. If an organoid is produced from patient's stem cells, it can be developed as a customized precision medical platform. Also, it is expected to be used for the purpose of regenerative medicine to reconstruct a damaged cardiac tissue such as myocardial infarction.

### BRIEF DESCRIPTION OF THE DRAWINGS

**FIG. 1A** illustrates the fabrication and analysis of a heart extracellular matrix (HEM) scaffold for cardiac organoid culture.
**FIG. 1B** illustrates the fabrication and analysis of a heart extracellular matrix (HEM) scaffold for cardiac organoid culture.
**FIG.2A** illustrates the fabrication and analysis of a heart extracellular matrix (HEM) scaffold for cardiac organoid culture.
**FIG.2B** illustrates the fabrication and analysis of a heart extracellular matrix (HEM) scaffold for cardiac organoid culture.
**FIG. 3** illustrates the analysis of an HEM scaffold proteome for cardiac organoid culture.
**FIG. 4A** shows the types of matrisome proteins of the HEM scaffold and the quantitative analysis result thereof.
**FIG. 4B** shows the types of matrisome proteins of the HEM scaffold and the quantitative analysis result thereof.
**FIG. 4C** shows the types of matrisome proteins of the HEM scaffold and the quantitative analysis result thereof.
**FIG. 5A** shows the result of analysis of non-matrisome proteins of the HEM scaffold.
**FIG. 5B** shows the result of analysis of non-matrisome proteins of the HEM scaffold.
**FIG. 5C** shows the result of analysis of non-matrisome proteins of the HEM scaffold.
**FIG. 6A** illustrates a comparative analysis and optimization selection of cardiac tissue decellularization methods.
**FIG. 6B** illustrates a comparative analysis and optimization selection of cardiac tissue decellularization methods.
**FIG. 6C** illustrates a comparative analysis and optimization selection of cardiac tissue decellularization methods.
**FIG. 6D** illustrates a comparative analysis and optimization selection of cardiac tissue decellularization methods.
**FIG. 7** illustrates a process of preparing a cardiac organoid using the HEM scaffold.
**FIG. 8A** shows the results of preparing a human induced pluripotent stem cell-derived cardiomyocyte.
**FIG. 8B** shows the results of preparing an HEM scaffold-based human cardiac organoid by organoid encapsulation.
**FIG. 9** shows the analysis of gene expression of the HEM scaffold-based human cardiac organoid prepared by organoid encapsulation.
**FIG. 10** shows the evaluation and analysis of myocardial differentiation degree of the HEM scaffold-based human cardiac organoid prepared by organoid encapsulation.
**FIG. 11** shows the evaluation and analysis of differentiation degree of the HEM scaffold-based human cardiac organoid prepared by organoid encapsulation.
**FIG. 12** shows the result of analysis of drug reactivity of the HEM scaffold-based human cardiac organoid prepared by organoid encapsulation.
**FIG. 13A** shows a method of preparing an HEM scaffold-based human cardiac organoid by encapsulating single cardiomyocytes.
**FIG. 13B** shows the result of analysis of gene expression of the HEM scaffold-based human cardiac organoid prepared by encapsulating single cardiomyocytes.
**FIG. 14** shows the result of culture of HEM scaffold-based vascular cells.
**FIG. 15A** shows the preparation of an HEM scaffold-based human cardiac organoid by encapsulating cardiomyocytes and two types of non-myocytes and the selection of optimized culture conditions.
**FIG. 15B** shows the preparation of an HEM scaffold-based human cardiac organoid by encapsulating cardiomyocytes and two types of non-myocytes and the selection of optimized culture conditions.
**FIG. 16A** shows the preparation of an advanced human cardiac organoid having a vascular structure by encapsulating cardiomyocytes and two types of non-myocytes, and a process and result of selecting a concentration of an HEM scaffold therefor.
**FIG. 16B** shows the preparation of an advanced human cardiac organoid having a vascular structure by encapsulating cardiomyocytes and two types of non-myocytes, and a process and result of selecting a concentration of an HEM scaffold therefor.
**FIG. 16C** shows the preparation of an advanced human cardiac organoid having a vascular structure by encapsulating cardiomyocytes and two types of non-myocytes, and a process and result of selecting a concentration of an HEM scaffold therefor.
**FIG. 17** shows the preparation of an advanced human cardiac organoid having a vascular structure by encapsulating cardiomyocytes and two types of non-myocytes, and a process and result of selecting a concentration of an HEM scaffold therefor.
**FIG. 18** shows the preparation of an advanced human cardiac organoid having a vascular structure by encapsulating cardiomyocytes and two types of non-myocytes, and a process of selecting a concentration of an HEM scaffold therefor.
**FIG. 19** shows a method and result of culturing a human cardiac organoid by using HEM encapsulation of cardiomyocytes and two types of non-myocytes and a microfluidic chip.
**FIG. 20** is a process and result of preparing an long QT syndrome (LQTS) patient's cardiac organoid by encapsulating LQTS cardiomyocytes and two types of non-myocytes and using the HEM scaffold.
**FIG. 21** shows the results of culture and analysis of the LQT patient's cardiac organoid prepared by using HEM encapsulation of cardiomyocytes and two types of non-myocytes and a microfluidic chip.

### MODE FOR CARRYING OUT THE INVENTION

In the present disclosure, a large amount of heart extracellular matrix-derived scaffolds were prepared through a series of chemical treatments on a porcine cardiac tissue and applied to culturing of a cardiac organoid. In order to culture a cardiac organoid, a decellularized scaffold was fabricated according to an optimal protocol by which a sufficient amount of extracellular matrix components can be retained. As a result of decellularization, it is possible to effectively remove cells in the cardiac tissue and also possible to preserve cardiac tissue-specific extracellular matrix components. It was confirmed that a hydrogel for three-dimensional organoid culture can be formed using the prepared heart extracellular matrix component.

In the present disclosure, a cardiac organoid was prepared by differentiating human induced pluripotent stem cells into cardiomyocytes, and three-dimensionally culturing the cells in the hydrogel made of the heart extracellular matrix. It was confirmed through the analysis of gene expression that a cardiac organoid cultured in a heart extracellular matrix-derived hydrogel scaffold exhibited an improvement in differentiation into cardiac tissues compared to commercially available scaffolds, Matrigel and a collagen hydrogel.

Also, it was observed that the cardiac organoid cultured in a heart extracellular matrix-derived hydrogel scaffold exhibited spontaneous beating and an excellent expression of cardiomyocyte-specific proteins and had noticeable cardiomyocyte-specific sarcomere structures compared to organoids cultured in Matrigel and a collagen hydrogel. Accordingly, it was confirmed that the heart extracellular matrix-derived scaffold can serve as a cardiac organoid culture scaffold and can be developed as a new functional culture platform that can replace the existing culture scaffolds.

The newly developed heart extracellular matrix-derived scaffold culture platform can be more simply prepared and more economical than existing expensive culture matrices and polymer scaffolds and thus is very advantageous for commercialization.

Hereinafter, Examples will be presented for understanding of the present disclosure. However, the following Examples are illustrative only for better understanding of the present disclosure but do not limit the present disclosure.

### Example 1: Preparation and analysis of heart extracellular matrix (HEM) scaffold for cardiac organoid culture

### Example 1-1. Preparation and analysis of heart extracellular matrix

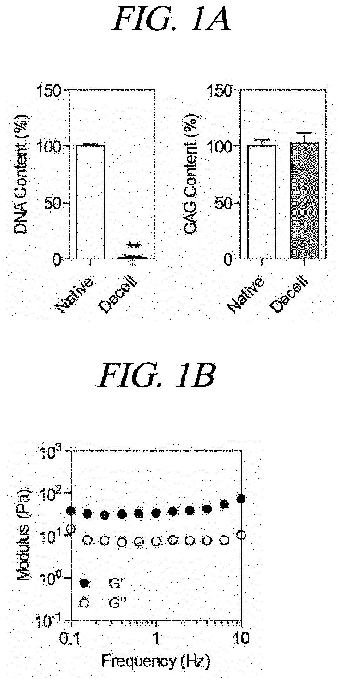

As shown in **FIG. 1****,** heart extracellular matrix (HEM) scaffolds were prepared from a porcine cardiac tissue through decellularization and properties thereof were analyzed.

Cardiac decellularization was performed by treatment with 1% sodium dodecyl sulfate (SDS) under refrigerated conditions for 24 hours and additional treatment with 1% Triton X-100 and 0.1% NH₄OH under refrigerated conditions for 6 hours. Extracellular matrix components produced through the decellularization were prepared in the form of powder through lyophilization. As a control group, a non-decellularized cardiac tissue was used.

As a result of quantitative analysis of DNAs and GAGs (glycosaminoglycans) to check whether cells were sufficiently removed from the prepared HEM scaffolds and whether the extracellular matrix components were well preserved, it was confirmed that after the decellularization, DNAs are sufficiently removed from all of the prepared HEM scaffolds and GAGs are present at a level similar to that of the actual cardiac tissue **(****FIG. 1A****).**

### Example 1-2. Preparation and analysis of scaffold composition

10 mg of lyophilized HEM-derived scaffold prepared in the form of powder through lyophilization (Lyophilized HEM) was treated with a 4 mg/ml pepsin solution (a solution of porcine gastric mucosa-derived pepsin powder (4 mg) dissolved in 0.02 M HCL (1 ml)) and then subjected to a solubilization at room temperature for 48 hours. Thereafter, it was adjusted to a neutral pH and electrolyte state of 1X PBS buffer by using 10X PBS and 1 M NaOH so as to be used for cell culture. Finally, hydrogel formation (gelation) was induced for 30 minutes at 37°C in an incubator.

When the properties of the hydrogel were measured by performing rheological analysis, it was confirmed that the prepared HEM-derived hydrogel scaffold has a stable polymer network structure and has mechanical properties suitable for organoid culture (FIG. 1 B).

Meanwhile, an HEM scaffold was prepared from a porcine cardiac tissue and properties thereof were analyzed.

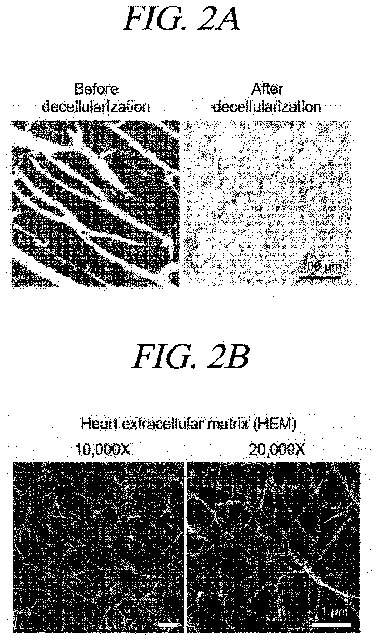

As a result, as can be seen from **FIG. 2****,** when the cardiac tissue before and after the decellularization was observed through H&E histological analysis, it was confirmed that most of the cell nuclei were removed, but the overall structure of the tissue was maintained **(****FIG. 2A****).** Further, as a result of scanning electron microscopy (SEM) analysis to check the internal structure of the hydrogel formed from the HEM-derived scaffold, It was confirmed that the inside of the HEM hydrogel is composed of nanofibrous extracellular matrix components, and, thus, the prepared hydrogel can provide a microenvironment suitable for growth of cardiac organoid **(****FIG. 2B****).**

### Test Example 1: Analysis of heart extracellular matrix

### Test Example 1-1. Analysis of heart extracellular matrix (HEM) scaffold proteome for cardiac organoid culture

In order to check an extracellular matrix component contained in the heart extracellular matrix-derived scaffold, proteomic analysis was conducted using a mass spectrometer.

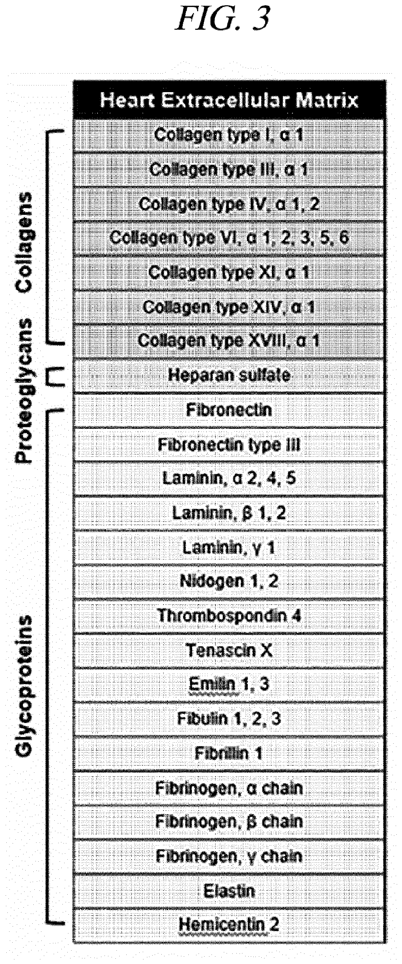

As a result, as shown in **FIG. 3****,** it was confirmed that many extracellular matrix components and glycoproteins, such as various collagens, proteoglycans and glycoproteins, are included in the heart extracellular matrix-derived scaffold. Accordingly, it is expected that these heart-specific components present in the actual cardiac tissue can promote cardiac organoid differentiation, structural development and functional enhancement.

### Test Example 1-2. Types of matrisome proteins of HEM scaffold and quantitative analysis

Matrisome proteins contained in HEM were detected through proteomic analysis using a mass spectrometer, classified by protein type, and relative quantitative analysis was conducted.

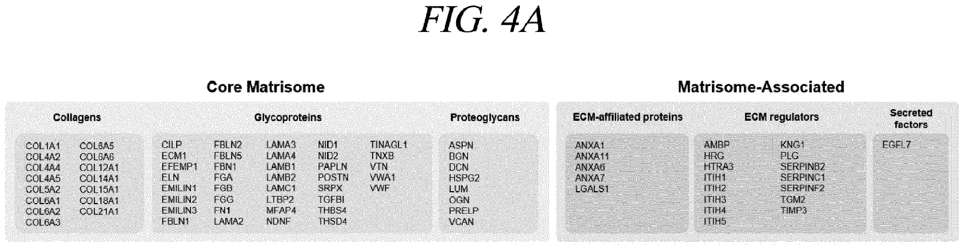

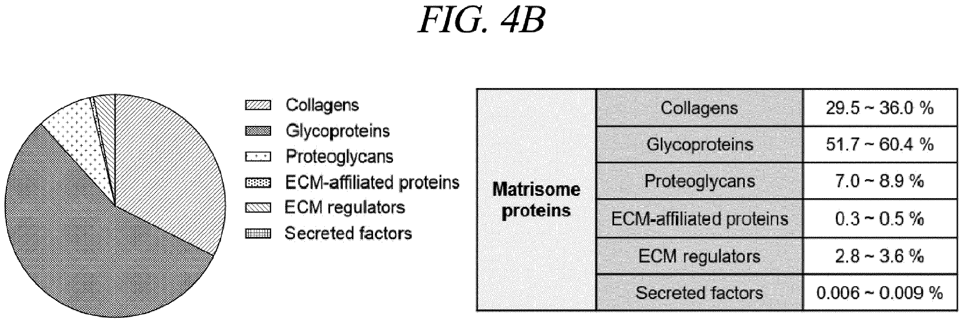

It was confirmed that various types of cardiac tissue proteins are present in the HEM, and it can be predicted that these components will have a positive effect on cardiac organoid culture **(****FIG. 4A** and **FIG. 4B****).**

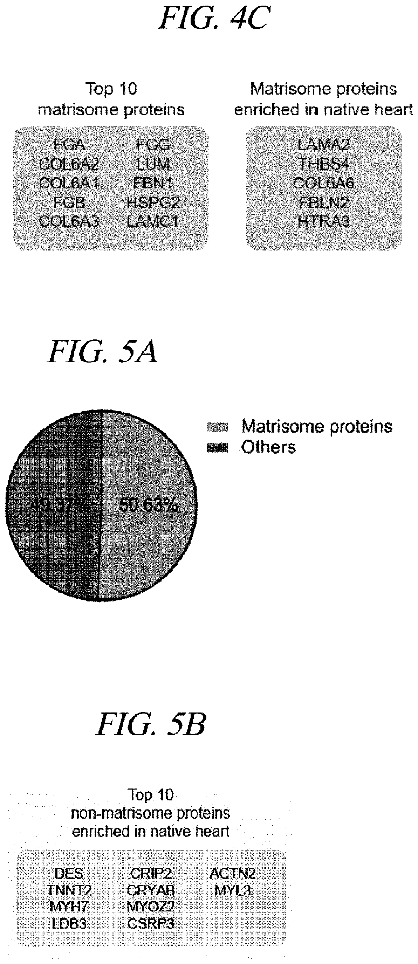

Also, the top ten most abundant matrisome proteins in the HEM through relative quantitative analysis of proteins were identified to be mostly collagens and fibrinogen subtypes. Further, it was confirmed that lumican, perlecan, and laminin (gamma 1) were also detected in large amounts. Furthermore, it was confirmed that various types of matrisome proteins (laminin (alpha 2), thrombospondin 4, collagen type 6 (alpha 6), fibulin 2, etc.) known as being present specifically in the cardiac tissue were detected in the HEM **(****FIG. 4C****).**

Therefore, it can be seen that compared to conventional culture systems based on heart-aspecific proteins (e.g., Matrigel) or single-component proteins (e.g., collagen and fibrin gel), the HEM hydrogel containing various cardiac tissue matrisome proteins provides a cardiac tissue-specific microenvironment helpful for cardiac organoid development and thus can help to produce more advanced mature cardiac organoids.

### Test Example 1-3. Analysis of non-matrisome protein of HEM scaffold

Non-matrisome proteins of the HEM scaffold of the present disclosure were analyzed.

As a result, it was confirmed that the HEM contained substantial amount of non-matrisome proteins as well as matrisome proteins **(****FIG. 5A****).** Also, it was confirmed that various non-matrisome proteins contained a lot in the actual cardiac tissue are contained in the HEM **(****FIG. 5B****).**

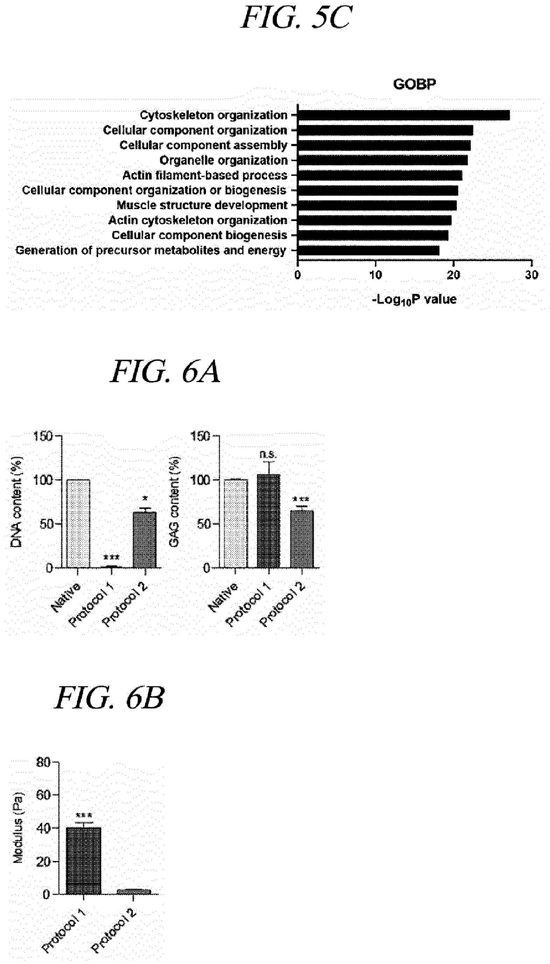

Meanwhile, the non-matrisome proteins of HEM scaffold were analyzed through a gene ontology biological process (GOBP) to check which biological processes are involved in the highly expressed proteins in the HEM. As a result, it was confirmed that the proteins highly expressed in the HEM are particularly related to formation of tissues such as cytoskeleton organization, cellular component organization, cellular component assembly, and organelle organization. **(****FIG. 5C****).**

Therefore, it can be seen that the use of the HEM scaffold containing these non-matrisome proteins makes it possible to culture a mature cardiac organoid.

### Test Example 1-4. Comparative analysis and optimization of cardiac tissue decellularization protocols

A test was conducted to compare the decellularization method of the present disclosure (Protocol 1) with another decellularization method (Protocol 2). In Protocol 2, unlike Protocol 1, treatment with 1% sodium dodecyl sulfate (SDS) was excluded and treatment with 1% Triton X-100 and 0.1% NH₄OH was conducted for only 6 hours.

As a result, when comparing the amount of DNAs remaining after the decellularization of the heart, it was confirmed that most of DNAs were removed from the decellularized heart matrix prepared by Protocol 1, but a significant amount of DNAs remained in the tissue prepared by Protocol 2. Also, when comparing the remaining extracellular matrix components through GAG quantitative analysis, it can be seen that GAG components were preserved after treatment of Protocol 1, whereas the amount of remaining GAG components showed a significant decrease after treatment of Protocol 2 **(****FIG. 6A****).**

Further, as a result of measuring mechanical properties (modulus) after preparing the hydrogel using the HEM, it was confirmed that the hydrogel prepared by Protocol 1 has a significantly high modulus **(****FIG. 6B****).**

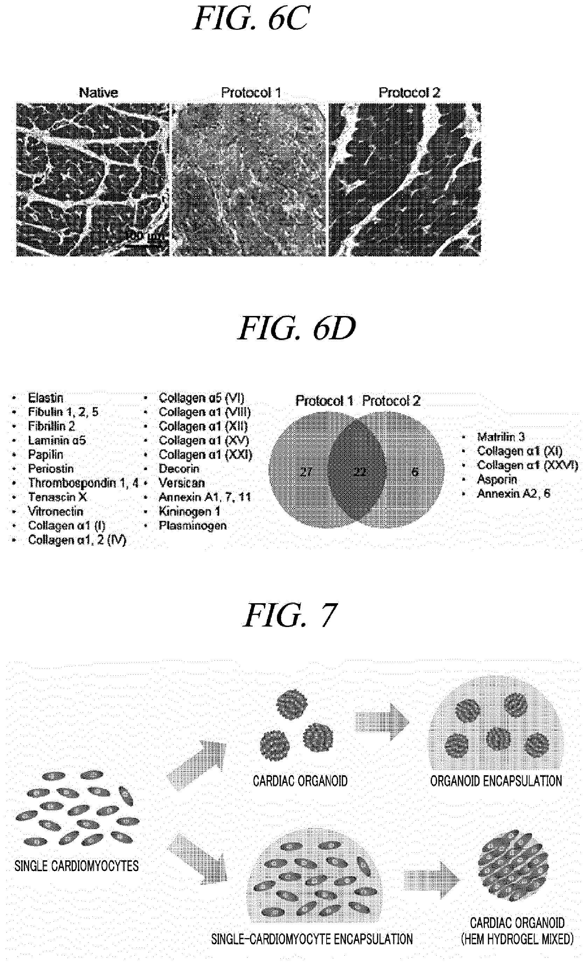

Furthermore, as a result of comparison by H&E histological analysis, it was confirmed that a large number of cells remained in the HEM prepared by Protocol 2 and maintained a shape similar to that of the actual cardiac tissue **(****FIG. 6C****).**

Meanwhile, as a result of proteomics analysis of the HEM prepared by each decellularization method, it was confirmed that Protocol 1 can preserve a greater number of extracellular matrix proteins than Protocol 2 **(****FIG. 6D****).**

Therefore, it can be seen that the decellularization method established in this study can preserve extracellular matrix proteins of cardiac tissue better than the other decellularization method and enables the production of a scaffold more suitable for cardiac organoid culture.

### Example 2: Preparation of cardiac organoid using HEM scaffold

Two methods for preparing a cardiac organoid using an HEM hydrogel and cardiomyocytes were conducted as shown in **FIG. 7****.**

Specifically, the first method is a method of preparing single cardiomyocytes in the form of a three-dimensional organoid by using a microwell and encapsulating and culturing the prepared cardiac organoid in the HEM hydrogel. The second method is a method of encapsulating single cardiomyocytes in the HEM hydrogel to prepare a cardiac organoid in which the HEM hydrogel is mixed between cells.

Hereinafter, it will be verified that each method can produce a cardiac organoid with enhanced myocardial differentiation by using an HEM hydrogel.

### Test Example 2: Verification of organoid encapsulation

### Test Example 2-1. Preparation of human induced pluripotent stem cell-derived cardiomyocyte and preparation of HEM scaffold-based human cardiac organoid by organoid encapsulation

Human induced pluripotent stem cells were induced to differentiate into cardiomyocytes, and the differentiated cardiomyocytes were prepared in the form of a three-dimensional organoid by using microwells. Thereafter, a cardiac organoid was three-dimensionally cultured in an HEM hydrogel (4 mg/ml) to produce a cardiac organoid with enhanced differentiation and maturity.

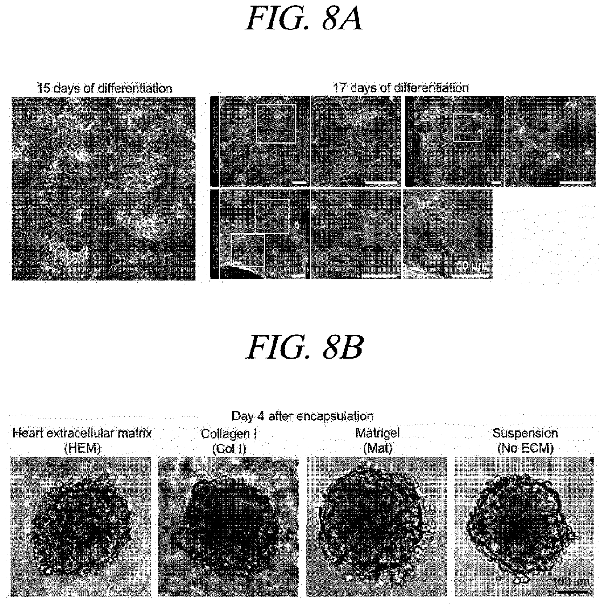

When differentiation of human induced pluripotent stem cells into cardiomyocytes was induced on a conventional culture plate, it was confirmed that the cells were well connected and spontaneously beating on the 15th day of differentiation. Also, the expression of α-actinin, a heart-specific protein, and the structure of sarcomere were also checked through immunostaining on the 17th day **(****FIG. 8A****).**

The differentiated cardiomyocytes were prepared in the form of a three-dimensional organoid by using microwells. The prepared organoid was subjected to three-dimensional culture in an HEM hydrogel. Groups cultured using a collagen hydrogel (Col I) and Matrigel (Mat), respectively, and a group float-cultured on a culture medium without a matrix (No ECM) were used as control groups. As a result, it was confirmed that the cardiac organoid cultured in the HEM hydrogel exhibited stronger beating than the organoids cultured in the control groups **(****FIG. 8B****).**

### Test Example 2-2. Analysis of gene expression of HEM scaffold-based human cardiac organoid prepared by organoid encapsulation

In order to check the enhancement of cardiac differentiation of a human cardiac organoid cultured three-dimensionally in an HEM hydrogel (4 mg/ml), qPCR analysis was conducted on the 7th day of culture. Groups cultured using a collagen hydrogel (Col I) and Matrigel (Mat), respectively, and a group float-cultured on a culture medium without a matrix (No ECM) were used as control groups.

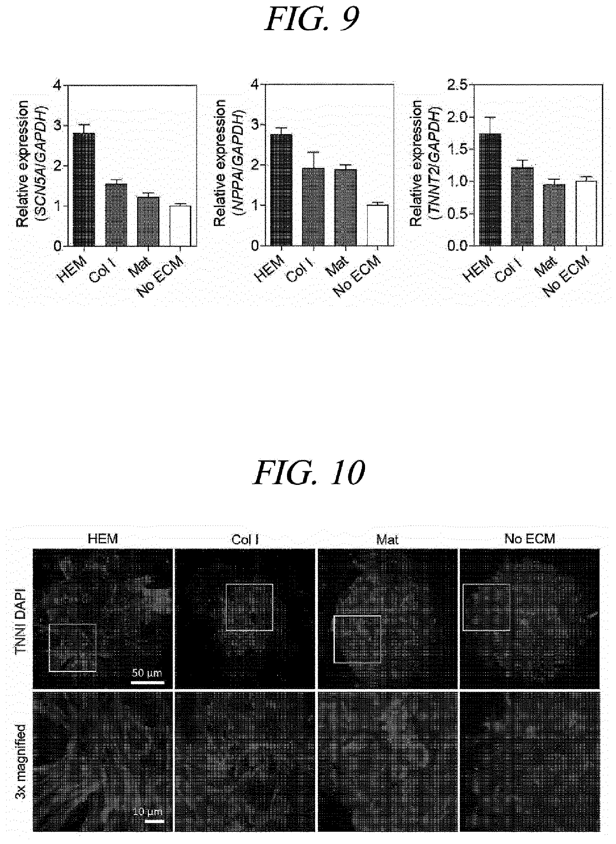

As a result, as shown in **FIG. 9****,** it was confirmed that the expression of myocardial-specific genes *TNNT2, NPPA* and *SCN5A* was further enhanced in the human cardiac organoid prepared using the HEM hydrogel than in the control groups.

Therefore, it was confirmed that the HEM hydrogel further promotes the myocardial differentiation of the human cardiac organoid than the conventional organoid culture scaffolds.

### Test Example 2-3. Evaluation and analysis of myocardial differentiation degree of HEM scaffold-based human cardiac organoid prepared by organoid encapsulation

In order to check the effect of the HEM hydrogel on the myocardial differentiation of the human cardiac organoid, immunostaining for Troponin I (TNNI), a myocardial-specific marker, was performed on the 7th day of culture. Groups cultured using a collagen hydrogel (Col I) and Matrigel (Mat), respectively, and a group float-cultured on a culture medium without a matrix (No ECM) were used as control groups.

As a result, as shown in **FIG. 10****,** it was confirmed that cardiomyocytes showing a clearly distinct sarcomere structure and higher expression of TNNI were observed in the human cardiac organoid prepared using the HEM hydrogel (4 mg/ml) compared to the control groups.

### Test Example 2-4. Evaluation and analysis of differentiation degree of HEM scaffold-based human cardiac organoid prepared by organoid encapsulation

In order to check the effect of the HEM hydrogel on the myocardial differentiation of the cardiac organoid, immunostaining for cardiomyocyte-specific markers α-actinin and cTnT was performed on the 7th day of culture, and the protein expression levels of the markers were compared. Groups cultured using a collagen hydrogel (Col I) and Matrigel (Mat), respectively, were used as control groups.

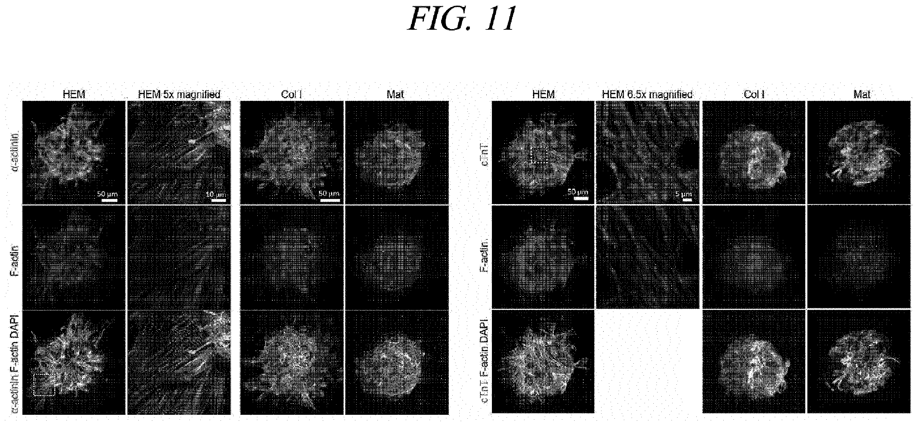

As a result, it was confirmed from **FIG. 11** that cardiomyocytes were present in a more mature form and a myocardial-specific sarcomere structure was more clearly observed in the human cardiac organoid prepared using the HEM hydrogel (4 mg/ml) than in the control groups.

### Test Example 2-5. Analysis of drug reactivity of HEM scaffold-based human cardiac organoid prepared by organoid encapsulation

In order to check the reactivity of the human cardiac organoid cultured in the HEM hydrogel (4 mg/ml) to drugs, treatment with two representative drugs (isoproterenol and propranolol) affecting the heart rate was performed on the 7th day of culture, and reaction of the organoid thereto was checked through calcium imaging.

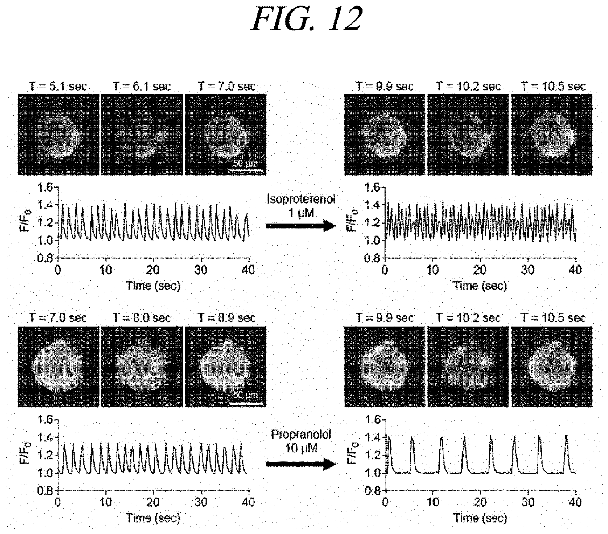

As a result, as can be seen from **FIG. 12****,** the beating of cardiac organoid is increased when treated with isoproterenol at a concentration of 1 µM, and the beating of cardiac organoid is decreased when treated with propranolol at a concentration of 10 µM.

Accordingly, it was confirmed that the human cardiac organoid cultured in the HEM hydrogel shows appropriate reactivity to the drugs, and, thus, it was verified that it can be used for evaluating cardiac responses to various drugs.

### Test Example 3: Verification of single-cardiomyocyte encapsulation

### Test Example 3-1. Method of preparing HEM scaffold-based human cardiac organoid by single-cardiomyocyte encapsulation and analysis of gene expression

Instead of the method of encapsulating the organoid in the HEM hydrogel, a method of preparing a human cardiac organoid by encapsulating human induced pluripotent stem cell-derived cardiomyocytes in the HEM hydrogel in a single-cell state was attempted.

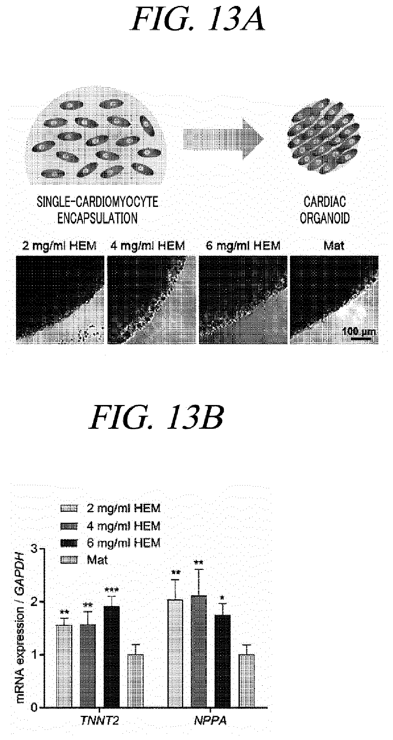

As a result, as the cells were cultured, the hydrogel contracted to form a hard mass of cardiac organoids. Cardiac organoids were prepared by using HEM hydrogels of various concentrations, and it was confirmed that a cardiac organoid having a relatively large size can produced compared to the method of encapsulating the organoid **(****FIG. 13A****).**

Further, in order to check the effect of the HEM hydrogel to enhance cardiac differentiation, qPCR analysis was conducted on the 7th day of culture, and a cardiac organoid cultured in Matrigel (Mat) was compared as a control group.

As a result, it was confirmed that the expression of myocardial-specific genes *TNNT2* and *NPPA* was further enhanced in the human cardiac organoid prepared using the HEM hydrogel than in the cardiac organoid cultured in Matrigel as the control group **(****FIG. 13B****).**

Therefore, it was confirmed that the HEM hydrogel further promotes the formation and myocardial differentiation of the human cardiac organoid through the encapsulation method of single cardiomyocytes.

### Test Example 3-2. Culture of HEM scaffold-based vascular cell

Since the formation of vascular structures inside an organoid is a very important factor in the growth, development and function of the organoid, it was checked whether it is possible to culture vascular cells in an HEM hydrogel for vascularization of a cardiac organoid.

Specifically, in order to determine whether the HEM hydrogel is suitable for culturing vascular endothelial cells, human umbilical vein endothelial cells (HUVECs) were encapsulated by a single-cell encapsulation method and cultured in HEM hydrogels of various concentrations.

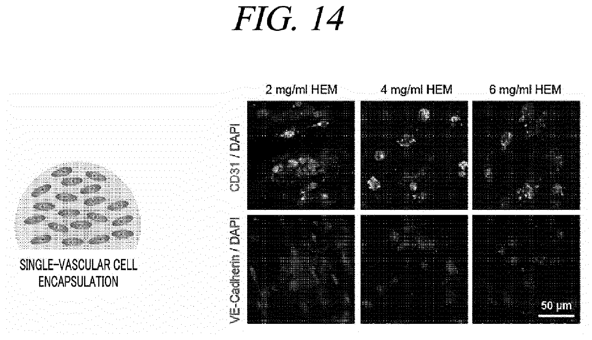

Through immunostaining for vascular markers CD31 and VE-Cadherin on the 7th day of culture, it can be seen from **FIG. 14** that although culture can be performed at any concentration, the expression levels of the vascular markers are high and cell spreading occurs well in the 2 mg/ml HEM hydrogel. Thus, it can be seen that 2 mg/ml is the optimal concentration for culture.

### Test Example 3-3. Preparation of HEM scaffold-based human cardiac organoid by encapsulating cardiomyocytes and two types of non-myocytes and selection of optimized culture conditions

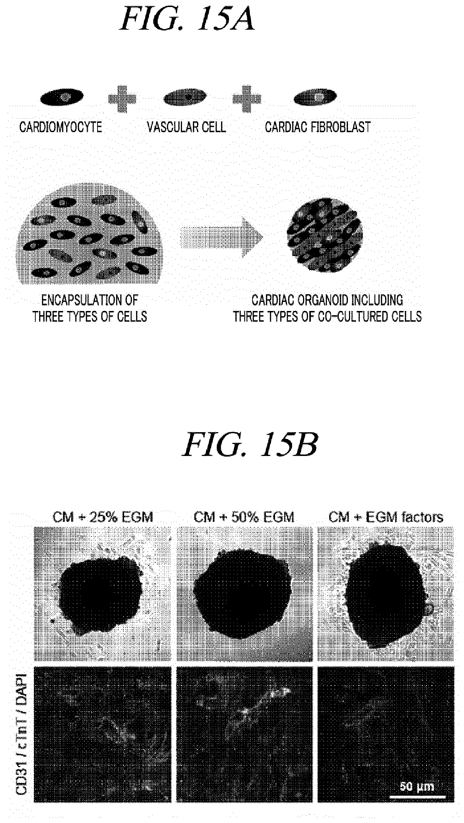

In order to fabricate a heart model with a structurally and functionally more advanced vascular structure, vascular endothelial cells and cardiac fibroblasts, which are non-myocytes present in the actual heart, were encapsulated together with cardiomyocytes by a single-cell encapsulation method in the HEM hydrogel to prepare a cardiac organoid including three types of co-cultured cells. HUVECs were used as vascular cells, and cardiac fibroblasts were differentiated from human induced pluripotent stem cells **(****FIG. 15A****).**

Specifically, in order to select co-culture medium conditions, a culture condition selection test was conducted by mixing a cardiomyocyte culture medium and a vascular cell culture medium in various ratios.

When the protein expression of the cardiomyocyte marker (cTnT) and the vascular cell marker (CD31) was checked through immunostaining on the 7th day of culture, it was confirmed that vascularization occurred best under the condition of CM (cardiomyocyte culture medium) + 50% EGM (vascular cell culture medium) without any problem with the expression of cardiomyocyte-specific proteins **(****FIG. 15B****).**

Therefore, subsequent preparations of co-cultured cardiac organoids were performed under the condition of CM + 50% EGM.

### Test Example 3-4. Preparation of advanced human cardiac organoid having vascular structure by encapsulating cardiomyocytes and two types of non-myocytes, and selection of concentration of HEM scaffold therefor

An advanced human cardiac organoid with a vascular structure was prepared by encapsulating cardiomyocytes and two types of non-myocytes and the concentration of an HEM scaffold therefore was selected.

Specifically, a total of 2 × 10⁵ (ratio 2:1:1) of 3 types of cells (cardiomyocytes, vascular cells, cardiac fibroblasts) were cultured by a single-cell encapsulation method in HEM hydrogels at concentrations of 2 mg/ml, 4 mg/ml and 6 mg/ml to prepare cardiac organoids.

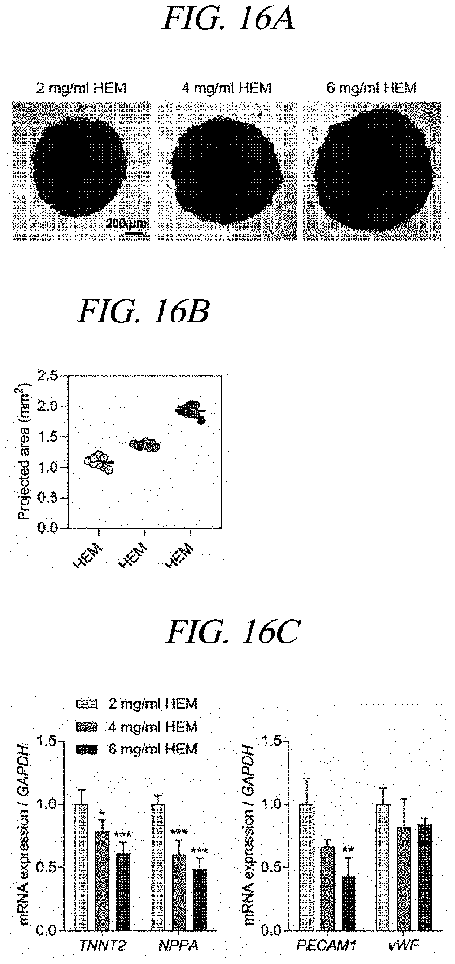

As a result, it was confirmed that spherical cardiac organoids were successfully produced through microscopic analysis on the 7th day of culture **(****FIG. 16A****).** Also, when comparing the sizes of cardiac organoids formed in the HEM hydrogels of different concentrations, respectively, on the 7th day of culture, it was confirmed that as the HEM concentration decreases, an organoid condenses to form a small organoid with a denser structure **(****Fig. 16B****).**

Further, as a result of comparing the mRNA expression level for each concentration of the HEM hydrogel through qPCR analysis on the 7th day of culture, when the expression of myocardial-specific genes *TNNT2* and *NPPA* and vascular-specific markers *PECAM1* and vWFwere checked, myocardial differentiation and vascular maturation were further enhanced in the low-concentration HEM hydrogel **(****FIG. 16C****).**

Therefore, it was confirmed that a cardiac organoid including three types of cells co-cultured in the 2 mg/ml HEM hydrogel was in the most condensed form with a dense structure and vascularization and myocardial differentiation were further enhanced.

Furthermore, on the 7th day of culture, the distribution of cardiomyocytes (cTnT), vascular cells (CD31) and cardiac fibroblasts (VIM, DDR2) was checked through immunostaining.

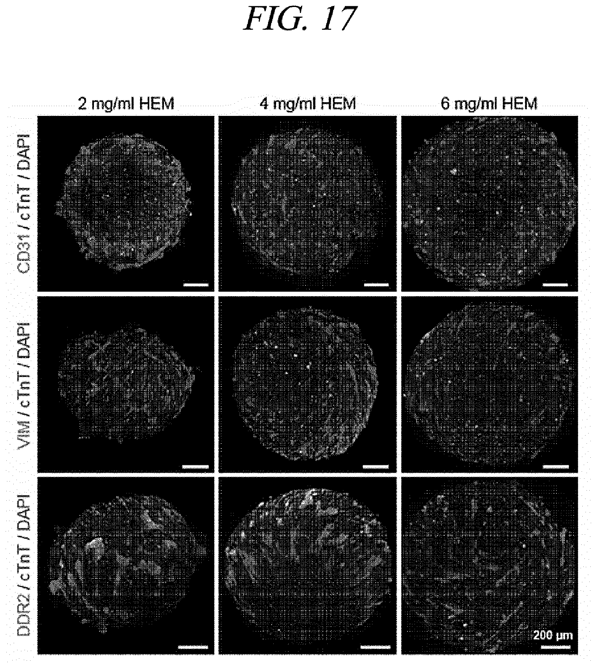

As a result, it can be seen from **FIG. 17** that as the HEM concentration decreases, an organoid condenses, and, thus, the cells are connected well and the expression levels of the respective markers are high.

Therefore, it was confirmed that vascularization and myocardial differentiation are further enhanced while a cardiac organoid is prepared in the most condensed form by co-culturing three types of cells in the 2 mg/ml HEM hydrogel.

### Test Example 3-5. Preparation of advanced human cardiac organoid having vascular structure by encapsulating cardiomyocytes and two types of non-myocytes, and selection of concentration of HEM scaffold therefor

In order to check the internal structure of cardiac organoids including three types of cells co-cultured in HEM hydrogels of various concentrations for 14 days, immunostaining was performed through histological analysis.

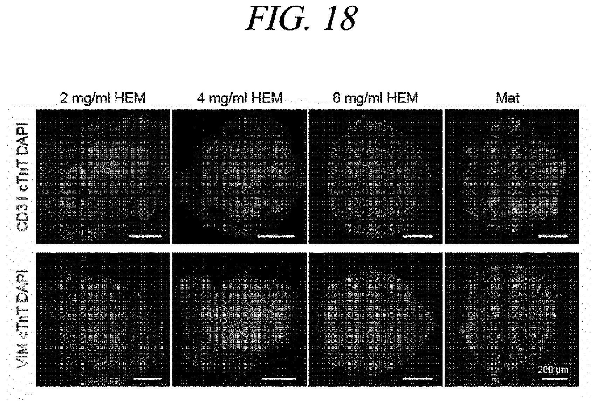

As a result of checking the cardiomyocyte marker cTnT, the vascular cell marker CD31 and the cardiac fibroblast marker VIM, it can be seen from **FIG. 18** that the cells are closely connected to form a dense structure in the HEM hydrogels at concentrations of 2 mg/ml and 4 mg/ml. It was confirmed that in the 6 mg/ml HEM group and the control group, Matrigel, the cells remained in the form of single cells and were not organized inside the organoid.

### Test Example 3-6. Culture of human cardiac organoid by using HEM encapsulation of cardiomyocytes and two types of non-myocytes and microfluidic chip

In order to fabricate a more advanced human heart model, a microfluidic chip capable of providing a microfluidic flow of culture medium that mimics the blood flow of the human body was used and a cardiac organoid was cultured using an HEM hydrogel in the chip. Matrigel (Mat) was used as a control group.

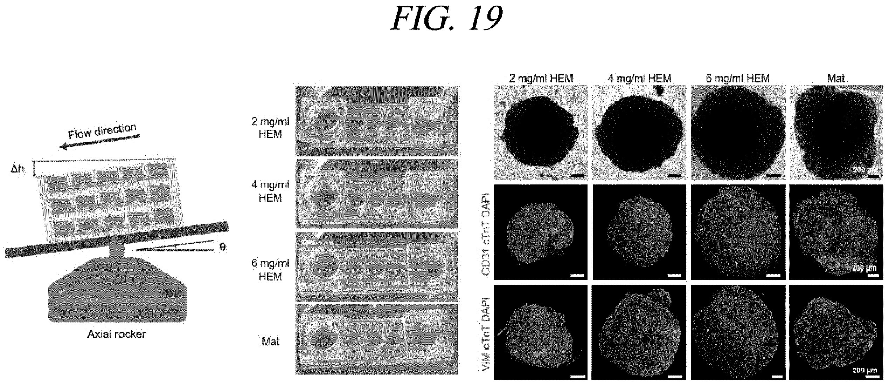

When the expression of the cardiomyocyte marker (cTnT), the vascular cell marker (CD31) and the cardiac fibroblast marker (VIM) were compared through immunostaining on the 14th day of culture, the expression levels of all the cell markers were the highest in the cardiac organoid in the 2 mg/ml HEM hydrogel, as shown in **FIG. 19****.**

Therefore, the present test confirmed that a more advanced human cardiac organoid including three types of co-cultured cells can be prepared by combining a microfluidic chip with an HEM hydrogel.

### Test Example 4: Confirmation of applicability of scaffold composition of the present disclosure

### Test Example 4-1. Preparation of LQTS patient's cardiac organoid by encapsulating cardiomyocytes and two types of non-myocytes and using HEM scaffold

A cardiac organoid was prepared by single-cell encapsulation using cardiomyocytes differentiated from induced pluripotent stem cells of a patient with LQTS. Specifically, it was prepared using a 2 mg/ml HEM hydrogel. In addition to cardiomyocytes, two types of non-cardiomyocytes (vascular cells and cardiac fibroblasts) were encapsulated together, and cardiac organoids were prepared using cardiomyocytes differentiated from stem cells of patients with different types of LQTS (LQT2 and LQT3).

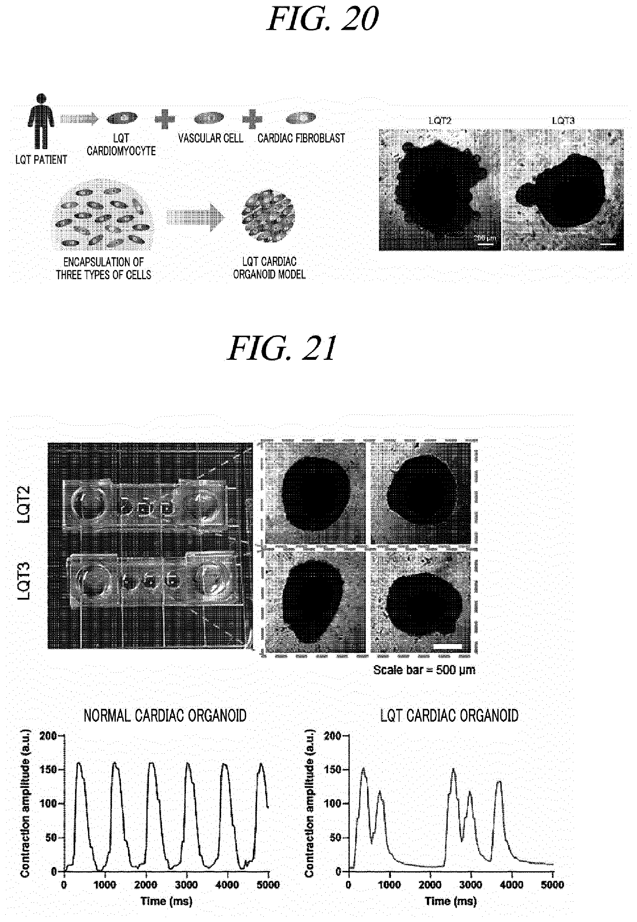

As can be seen from FIG. 20, it was confirmed that LQTS cardiac organoids were formed in the HEM hydrogels and cultured successfully through optical microscopic analysis on the 14th day of culture.

Accordingly, it can be seen that a human cardiac disease organoid model can be fabricated using an HEM hydrogel, and, thus, the HEM hydrogel can be applied as an effective organoid culture matrix for in vitro disease modeling.

### Test Example 4-2. Culture and analysis of LQTS patient's cardiac organoid prepared by using HEM encapsulation of cardiomyocytes and two types of non-myocytes and microfluidic chip

As in Test Example 4-1, vascular endothelial cells, cardiac fibroblasts and LQTS patient-derived induced pluripotent stem cell-derived cardiomyocytes were co-cultured in a 2 mg/ml HEM hydrogel, applied into a microfluidic chip and dynamically cultured with a microfluidic flow of culture medium to prepare an LQTS cardiac organoid.

As can be seen from **FIG. 21****,** it was confirmed that a normal cardiac organoid exhibited regular beating, whereas the LQTS organoid exhibited Irregular beating through analysis of heartbeat of the cardiac organoids on the 14th day of culture.

The present test confirmed that the LQTS organoid prepared by using the HEM hydrogel has the characteristics of the disease.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure.

## Claims

1. A scaffold composition containing a heart extracellular matrix (HEM).

2. The scaffold composition of Claim 1,
wherein the heart extracellular matrix is contained in a concentration of from 0.01 mg/mL to 10 mg/mL.

3. The scaffold composition of Claim 1,
wherein the composition has an elastic modulus of from 1 Pa to 150 Pa at 0.1 Hz to 10 Hz.

4. A method of preparing a scaffold composition, comprising:
a process (a) of decellularizing an isolated cardiac tissue to prepare a decellularized cardiac tissue; and
a process (b) of drying the decellularized cardiac tissue to prepare a heart extracellular matrix (HEM).

5. The method of preparing a scaffold composition of Claim 4,
wherein in the process (a), the isolated cardiac tissue is subjected to treatment with 0.1% to 2% sodium dodecyl sulfate (SDS) at 1°C to 10°C for 12 hours to 36 hours and decellularized with stirring in a decellularization solution.

6. The method of preparing a scaffold composition of Claim 5,
wherein the decellularization solution includes 0.1% to 5% Triton X-100 and 0.01% to 0.5% ammonium hydroxide.

7. The method of preparing a scaffold composition of Claim 4,
wherein through the decellularization in the process (a), 95% to 99.9% of cardiac tissue cells are removed.

8. The method of preparing a scaffold composition of Claim 4, further comprising:
a process of adjusting the HEM to be contained in a concentration of from 0.01 mg/mL to 10 mg/mL after the process (b).

9. A method of culturing a cardiac organoid in the scaffold composition of Claim 1 or a scaffold composition prepared by the preparation method of Claim 4.
